# FASCICULE DE BREVET EUROPEEN

(11) **EP 2 618 999 B1**
(45) Date de publication et mention de la délivrance du brevet: **14.01.2015**
(21) Numéro de dépôt: 11758497.9
(22) Date de dépôt: 02.09.2011
(51) Int. Cl.: B32B 5/14

(54) **STRATIFIE ELASTIQUE A RESISTANCE AUX EFFORTS RENFORCEE.**
NACHGIEBIGES LAMINAT MIT ERHÖHTER SPANNUNGSFESTIGKEIT
RESILIENT LAMINATE HAVING INCREASED STRENGTH AGAINST STRESSES

(30) Priorité: 23.09.2010 FR 1003776
(43) Date de publication de la demande: 31.07.2013
(73) Titulaire: Aplix, 44850 Le Cellier (FR)
(72) Inventeur: MARCHE, Thierry, F-44450 La Chapelle Basse Mer (FR); MOINARD, Nathalie, F-44300 Nantes (FR)
(74) Mandataire: Eidelsberg, Olivier Nathan
(86) Numéro de dépôt international: PCT/FR2011/000487
(87) Numéro de publication internationale: WO 2012/038613

(56) Documents cités:
- US-A1- 2005 060 849
- US-A1- 2007 154 683
- US-B1- 6 221 483
- US-B1- 6 245 401
- DATABASE WPI Week 199502 Thomson Scientific, London, GB; AN 1995-009314 XP002641187, & JP 6 293067 A (NITTO DENKO CORP) 21 octobre 1994 (1994-10-21)

## Description

La présente invention se rapporte à un stratifié comportant au moins un film élastique fixé, notamment par l'intermédiaire d'adhésif, notamment de colle, à au moins une couche de non-tissé, et notamment deux couches de non-tissé respectivement supérieure et inférieure. Ce genre de stratifié est utilisé en particulier dans des applications dans le domaine des vêtements, notamment jetables, tels que des couches-culottes et des dispositifs contre l'incontinence pour adulte, ou dans le domaine médical sous la forme de bandage élastique. Dans les couches-culottes, les stratifiés sont pratiquement utilisés dans une partie formant la ceinture autour de la taille des bébés. Dans le cas d'une application particulièrement avantageuse pour les couches-culottes, ces stratifiés sont utilisés pour réaliser les rabats élastiques qui garantissent que la couche-culotte est maintenue fermement sur le bébé, notamment élastiquement, le rabat portant par exemple des éléments mâles d'un autoagrippant, notamment des crochets destinés par exemple à coopérer avec des boucles d'une partie centrale de la ceinture de la couche culotte.

On connaît déjà dans de nombreux documents de l'art antérieur des stratifiés du genre mentionné ci-dessus. On souhaiterait avoir à disposition des stratifiés de ce genre que l'on puisse obtenir de la manière la plus simple possible, notamment adaptés à une fabrication à très grande échelle et qui soient, en outre, très résistants au cours du temps aux efforts qu'ils peuvent subir pendant leur utilisation, notamment dans le cadre de la fabrication d'une couche-culotte.

Suivant l'invention, le stratifié comportant un empilement dans une direction verticale d'au moins une couche de non-tissé et d'une strate d'une pièce ou d'un seul tenant fixée à la au moins une couche de non-tissé, notamment par un adhésif tel que de la colle, la strate ayant une première face ou face inférieure et une deuxième face ou face supérieure entre lesquelles s'étendent au moins une zone élastique en forme de film et au moins une zone dite rigide en forme de film en un matériau moins élastique que le matériau de la zone élastique, notamment une zone en un matériau non élastique, la strate étant obtenue par coextrusion des au moins deux films élastique et rigide, de sorte qu'il est formé entre eux une interface, est caractérisé en ce que, vu en coupe,
- la courbe ou ligne formée par l'interface entre les deux films s'étend d'un point de la face inférieure (dit point inférieur) à un point de la face supérieure (dit point supérieur) mutuellement décalés dans la direction horizontale, la droite diagonale passant par ces deux points étant incliné par rapport à la verticale à la strate ;
- on définit le point sommet de la courbe du côté du film rigide comme étant le point le plus éloigné de la droite diagonale du côté du film rigide, la distance à la droite diagonale étant mesurée perpendiculairement à celle ci, le point sommet étant confondu avec le point inférieur dans le cas limite où la courbe ne dépasse pas la diagonale du côté du film rigide et on définit le point creux de la courbe du côté du film élastique comme étant le point le plus éloigné de la droite diagonale du côté du film élastique, la distance à la droite diagonale étant mesurée perpendiculairement à celle ci, le point creux étant confondu avec le point supérieur dans le cas limite où la courbe ne dépasse pas la diagonale du côté du film élastique ;
- au moins l'un des points sommet et creux étant distincts respectivement des points inférieur et supérieur ;
- la droite passant par le point inférieur et le point sommet et/ou la droite passant par le point supérieur et le point creux faisant un angle avec la droite passant par le point creux et le point sommet ; et
- au moins l'une de la droite point inférieur-point sommet, de la droite point sommet-point creux et de la droite point creux-point supérieur est inclinée respectivement par rapport à la direction verticale, par rapport à la direction horizontale et par rapport à la direction verticale.

Suivant un premier mode de réalisation, le point creux est confondu avec le point supérieur et le point inférieur est distinct du point sommet.

Suivant un second mode de réalisation, le point sommet est confondu avec le point inférieur et le point supérieur est distinct du point creux.

Suivant un troisième mode de réalisation, le point sommet est distinct du point inférieur et le point supérieur est distinct du point creux.

Suivant un mode de réalisation, le point inférieur et le point sommet sont distincts, la droite point inférieur-point sommet est inclinée par rapport à la verticale et la droite point creux-point sommet est sensiblement horizontale.

Suivant un autre mode de réalisation, le point inférieur et le point sommet sont distincts, la droite point inférieur point sommet est sensiblement verticale et la droite point sommet-point creux est inclinée par rapport à l'horizontale.

Suivant un autre mode de réalisation, le point inférieur et le point sommet sont distincts, la droite point inférieur-point sommet est inclinée par rapport à la verticale et la droite point sommet-point creux est inclinée par rapport à l'horizontale.

Suivant un mode de réalisation particulièrement préféré, la courbe comporte entre la face inférieure et le point sommet un segment inférieur sensiblement rectiligne, notamment rectiligne.

En particulier la courbe comporte un segment inférieur sensiblement rectiligne, notamment rectiligne qui s'étend de la face inférieure jusqu'au point sommet.

Suivant un mode de réalisation particulièrement préféré, la courbe comporte entre le point sommet et le point creux un segment intermédiaire sensiblement rectiligne, notamment rectiligne.

En particulier, la courbe comporte un segment intermédiaire qui s'étend du point sommet jusqu'au point creux.

De préférence, l'extension dans la direction horizontale de la droite point inférieur-point sommet est inférieure à l'extension dans la direction horizontale de la droite point sommet-point creux.

Suivant un mode de réalisation préféré de l'invention, le segment inférieur sensiblement rectiligne et le segment point sommet-point creux sont en succession, notamment en se rejoignant au niveau du point sommet formant coude.

De préférence, le point creux est distinct du point supérieur; et la droite point supérieur point creux fait un angle avec la droite point sommet-point creux.

En particulier, la droite point supérieur-point creux est verticale.

Suivant un autre mode de réalisation, la droite point supérieur-point creux est inclinée par rapport à la verticale.

Suivant un mode de réalisation préféré, la courbe comporte un segment rectiligne qui s'étend entre le point supérieur et le point creux, notamment du point supérieur jusqu'au point creux.

Suivant un mode de réalisation préféré, la courbe comporte un segment sensiblement rectiligne, notamment rectiligne, qui s'étend entre le point sommet et le point creux, notamment du point sommet jusqu'au point creux.

Suivant un mode de réalisation préféré de l'invention, le tronçon de la courbe qui s'étend entre le point sommet et le point creux comporte un point d'inflexion.

Suivant un mode de réalisation préféré de l'invention, le point sommet se trouve à une hauteur par rapport à la face inférieure de la strate qui correspond à au moins 80 % de la hauteur totale ou épaisseur de la strate, telle que mesurée le long de la verticale passant par ce point.

Ainsi, la partie rigide forme au dessus du point d'inflexion ou de la zone d'inflexion segment une sorte de languette mince au-dessus de la partie élastique dont l'épaisseur la plus petite correspond à moins de 20 % de l'épaisseur de la strate.

On obtient ainsi une jonction entre la partie rigide et la partie élastique dans la strate qui assure une excellente résistance de la liaison partie rigide-partie élastique de la strate, notamment qui résiste bien aux tractions qu'elle peut subir, notamment en utilisation lorsque le stratifié est étiré, par exemple lorsqu'il est intégré dans une couche culotte, notamment au niveau de la fermeture de la couche au niveau de la ceinture, par exemple lorsqu'il est intégré dans une patte en forme d'oreille pouvant comporter des crochets que l'on rabat sur une partie centrale de la ceinture qui elle comporte des boucles destinées à coopérer avec les crochets pour fermer la couche.

Suivant un mode de réalisation préféré de l'invention, la dimension totale en largeur (dans le sens CD) de la partie rigide qui s'étend au-dessus de la partie élastique est inférieure à la largeur de la partie élastique.

Suivant un mode de réalisation préféré de l'invention, la strate comporte deux parties rigides de part et d'autre de la partie élastique et les deux parties formant languettes minces respectives des deux parties rigides s'étendent chacune au-dessus de la partie élastique en restant à distance l'une de l'autre au niveau de leur extrémité.

Suivant un mode de réalisation particulièrement préféré de l'invention, les deux languettes minces respectives des deux parties rigides de part et d'autre de la partie élastique se rejoignent à leur extrémité suivant une interface de languette.

A titre d'exemple, on décrit maintenant un mode de réalisation préféré de l'invention en se reportant au dessin, dans lequel :
- la Figure 1: est une vue en coupe CD, c'est-à-dire dans le plan transversalement à la direction machine (MD) et comportant la direction CD et la direction verticale, d'un stratifié suivant l'invention ;
- la Figure 2: est une vue en coupe d'un autre mode de réalisation d'un stratifié suivant l'invention ;
- la Figure 3: est une vue en coupe d'encore un autre mode de réalisation d'un stratifié suivant l'invention ;
- la Figure 4: est une vue en coupe d'encore un autre mode de réalisation d'un stratifié suivant l'invention ;
- la Figure 5: représente une vue en perspective d'une installation de production d'un stratifié suivant l'invention, notamment des stratifiés des figures précédentes :
- la Figure 6: est un schéma représentant un échantillon de matériau élastique ou élastomérique préparé pour en déterminer le SET ;
- la Figure 7: représente la forme de la courbe d'hystérésis obtenue lors de la détermination du SET d'un matériau, notamment élastique ou élastomérique ;
- La Figure 8: représente une vue en coupe transversale de d'encore un autre mode de réalisation de la strate après sa formation par extrusion et avant d'être assemblée à une ou deux couches de non tissé ;
- La Figure 9: représente des exemples de courbes donnant l'étirement en fonction de la force pour le test de caractère étirable relatif ;
- Les Figure 10 à 15: représentent chacune encore un autre mode de réalisation d'une strate suivant l'invention ; et

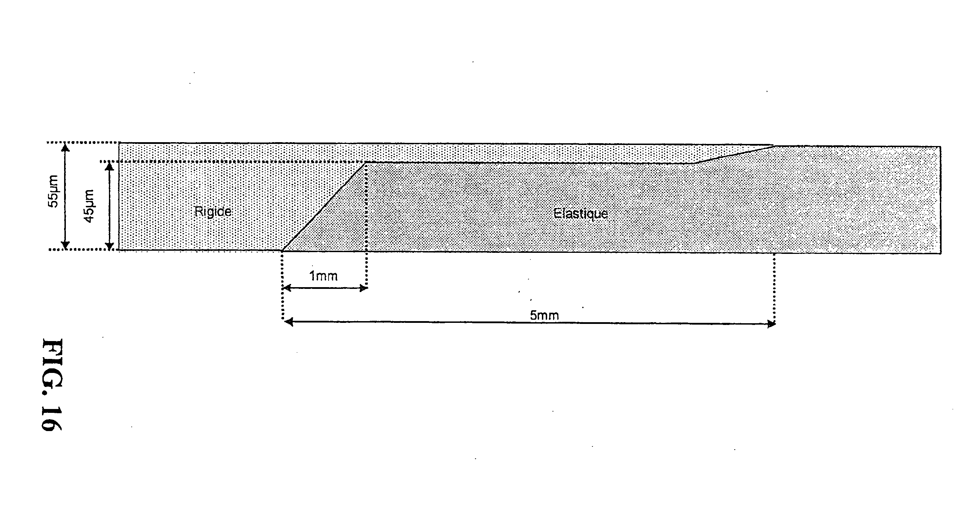

- La Figure 16: est une photographie d'une coupe d'un stratifié suivant l'invention.

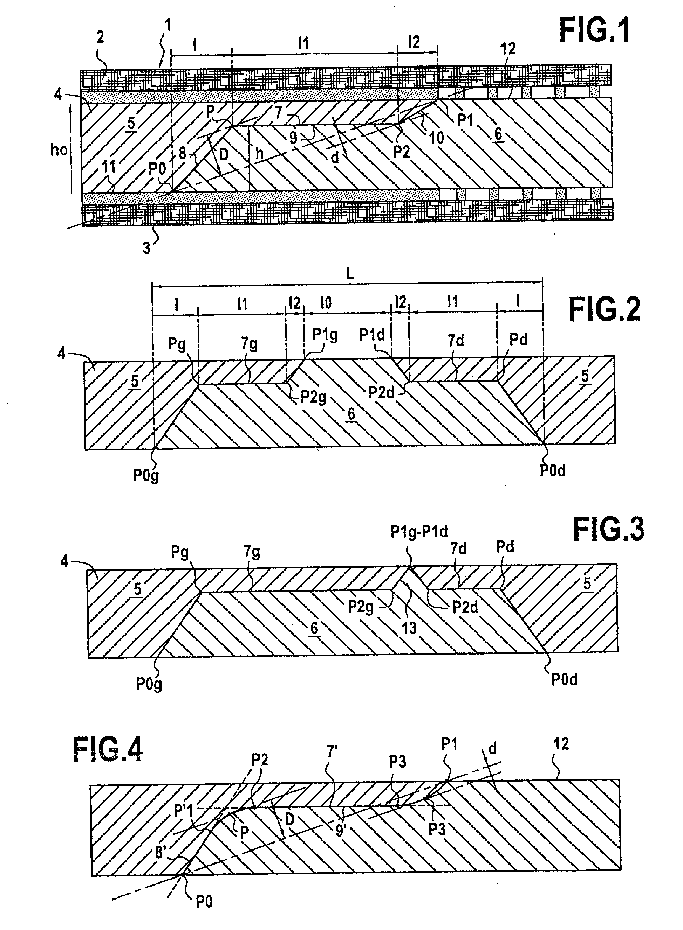

A la Figure 1, il est représenté un stratifié 1 comportant une couche 2 de non-tissé supérieure, une couche 3 de non-tissé inférieure et une strate 4 intermédiaire prise en sandwich entre les deux couches de non-tissé. Cette strate 4 comporte une partie 5 rigide et une partie 6 élastique. Le stratifié se poursuit vers la droite et, notamment, soit sous une forme identique, soit en comportant ensuite une autre partie 5 comme représentée à la Figure 2.

La partie 5 est dite rigide car moins élastique que la partie élastique. La formation de la strate 4 est réalisée par coextrusion de deux films correspondants aux parties respectivement. 5 et 6. La coextrusion des deux films pour la formation de la strate 4 est réglée de sorte qu'il est formé entre eux une interface défini par une courbe 7.

Cette courbe 7 comporte un premier segment 8 rectiligne partant de la face 11 inférieure de la strate, suivi d'un deuxième segment 9 rectiligne, lui-même suivi d'un troisième segment 10 rectiligne d'extrémité. Le premier segment 8 rectiligne s'étend à partir du point P0 de la face 11 inférieure de la strate en direction de la partie 6 élastique jusqu'à un point P sommet(ici le point sommet P est distinct du inférieur P0).

Le point P est le point qui se trouve, du côté de la diagonale où se trouve le film rigide 5, à la plus grande distance de la droite diagonale P0P1 tel que mesurée perpendiculairement à la droite diagonale P0P1.

Ce segment 8 rectiligne est incliné d'un angle de 30°par rapport à la direction verticale. A partir du point P sommet, la courbe 7 comporte le deuxième segment 9 rectiligne. Ce segment 9 est incliné par rapport à la direction verticale d'un angle supérieur à l'angle dont est incliné le segment 8 par rapport à la verticale et notamment d'un angle de 90°, tel que représenté à la Figure 1. Cet angle pourrait cependant ne pas être de 90°, mais être inférieur à 90°, par exemple être compris entre 70 et 80°.

Le segment 9. intermédiaire s'étend du point sommet P jusqu'à un point creux P2 qui est le point de la courbe du côté de la diagonale où se trouve le film élastique 6, à la plus grande distance de la droite diagonale P0P1 tel que mesurée perpendiculairement à la droite diagonale P0P1. Dans ce mode de réalisation, le point creux P2 et le point supérieur P1 sont distincts.

Ainsi, à la suite du segment 9 intermédiaire, le segment 10 d'extrémité s'étend du point P2 creux jusqu'au point P1 de la face 12 supérieure de la strate.

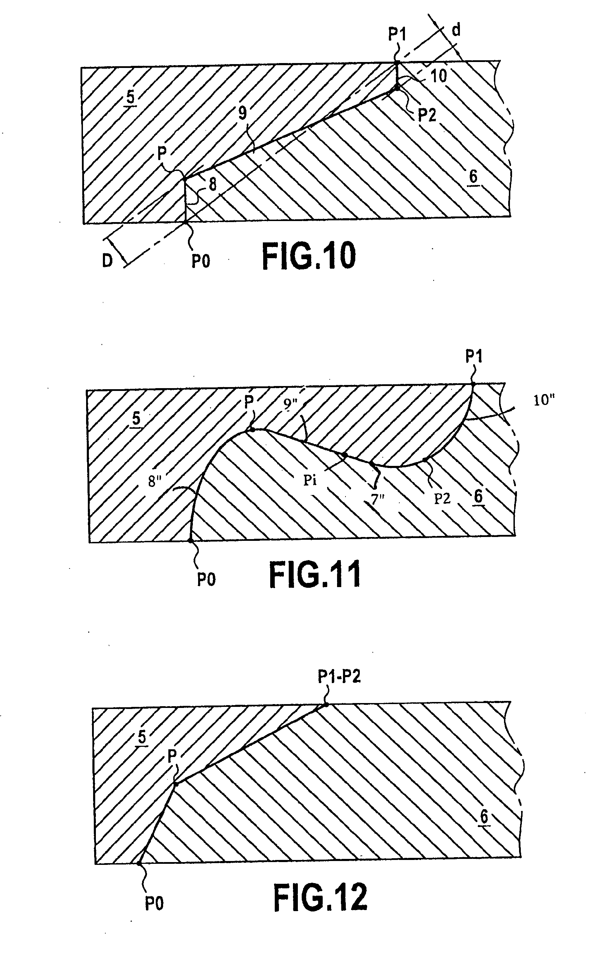

A la place des deux segments 9 et 10, on pourrait, par exemple, n'avoir qu'un seul segment sensiblement rectiligne qui s'étend à partir du point P jusqu'au point P1, comme représenté à la figure 12. dans ce cas, le point supérieur P1 et le point creux P2 sont confondus, la courbe ne dépassant jamais la diagonale P0P1 du côté du film élastique.

En outre, dans la réalité ou dans le détail plus microscopique, il peut arriver que les parties intermédiaires entre les segments 8, 9 et 10 ne soient pas aussi rectilignes que représenté et en particulier les parties intermédiaires entre les segments rectilignes 8 et 9 et entre les segments rectilignes 9 et 10 peuvent être légèrement incurvées comme représenté à la Figure 4. La courbe peut avoir par exemple un premier segment sensiblement rectiligne, par exemple 8' à la Figure 4, qui s'étend jusqu'au point P1' et un segment rectiligne 9', qui s'étend entre le point P2 et le point P3. Entre le point P1' et le point P2 de la courbe 7', il y a un segment incurvé. De même à partir du point P3 j'usqu' à la surface supérieure 12 de la strate, la courbe 7' a une forme incurvée. Dans le cas de la Figure 4, pour définir le point P, on prend le point à la plus grande distance D de la diagonale P0P1 et pour le point P2 le point à la plus grande distance d de la diagonale du côté de l'élastique.

La strate est fixée de part et d'autre aux deux non-tissés 2 et 3, notamment comme représenté par des couches de colle, les couches de colle étant continues au-dessus et en dessous de la partie rigide 5 et sous forme de points ou de lignes discontinues au-dessus et en dessous de la partie élastique. Ainsi, une fois le stratifié activé, c'est-à-dire étiré une première fois, par exemple en le faisant passer entre deux rouleaux dentés, les non-tissés vont s'étirer puis se relâcher et pouvoir ainsi suivre l'élasticité de la partie 6. Suivant également une autre possibilité, on peut utiliser des non-tissés étirables qui n'ont pas besoin d'être activés et qui vont pouvoir également suivre l'élasticité de l'élastique 6 pour assurer une élasticité au stratifié 1 dans son ensemble.

Le point P se trouve à une hauteur h qui correspond à au moins 80 % de la hauteur h0 totale ou épaisseur de la strate. En particulier, h peut être comprise entre 80 et 95 % de la hauteur h0. Ainsi, il est formé au-dessus du film 6 élastique au niveau du deuxième segment 9 rectiligne intermédiaire une languette de matière moins élastique ou rigide qui est très mince (entre moins de 20 % et 5 %, voire moins, de l'épaisseur totale de la strate). Cette partie en languette est plus allongée que la partie en biseau correspondant au segment 8 rectiligne. En particulier, la largeur 11 sur laquelle s'étend le segment 9 rectiligne intermédiaire est supérieure à la largeur 1 sur laquelle s'étend dans la direction horizontale le segment 8 rectiligne, et notamment environ trois à cinq fois plus grand.

Suivant un mode de réalisation comme représenté à la Figure 1, la couche de colle est continue entre le ou chaque non-tissé et la partie rigide jusqu'à environ au niveau du point P1 en haut et en bas, notamment comme représenté à la figure 1, jusqu'à un peu au delà du point P1. Elle est ensuite discontinue au dessus et en dessous de la partie élastique. Suivant un autre mode de réalisation possible, on pourrait faire en sorte que la couche de colle soit continue jusqu'au point P0 puis discontinue à partir du point P0 en haut et en bas sur toute la largeur où se trouve la partie élastique 6.

On pourrait également choisir de réaliser les couches de colle supérieure et inférieure continue jusqu'à un point compris entre P et P1, notamment jusqu'à P1. On peut également faire s'étendre la colle en haut et en bas jusqu'à des niveaux différents, par exemple au dessus ou en haut jusqu'à légèrement au delà du point P1 et en dessous ou en bas jusqu'à une moindre distance, par exemple jusqu'à avant P1, voire jusqu'à P0 ou même avant P0.

A la Figure 2, il est représenté un autre mode de réalisation dans lequel deux parties 5 se trouvent de part et d'autre de la partie 6 élastique de la strate 4. Les deux courbes respectives 7g et 7d (g et d pour gauche et droite) ont des formes sensiblement identiques en étant cependant inversées comme dans un miroir. Les deux points P1g et P1d sont à distance l'un de l'autre au niveau de la face 12 supérieure d'une distance 10.

Suivant un mode de réalisation préféré de l'invention, les deux points P1g et P1d comme représentés à la Figure 3 se confondent et les deux languettes se rejoignent en une interface 13. De préférence, comme représenté, l'une des languettes (celle de gauche dans l'exemple de la figure 3) s'étend sur une plus grande distance horizontale que l'autre languette (celle de droite dans l'exemple de la figure 3).

A la figure 10, il est représenté un mode de réalisation comportant un segment inférieur vertical qui s'étend à partir de la face inférieure jusqu'au point P sommet, suivi d'un segment rectiligne intermédiaire entre les points P sommet et P2 creux incliné par rapport à la verticale et par rapport à l'horizontale. Enfin, un segment supérieur vertical s'étend à partir du point creux P2 jusqu'à la face supérieure P1.

A la figure 11, il est représenté un cas ne comportant pas de segment rectiligne. A partir de la face inférieure P0 et jusqu'au point P la courbe 7" comporte un tronçon 8" incurvé dont la concavité est tournée vers la diagonale P0P1, la droite P0P étant incliné par rapport à la verticale. De même, la courbe comporte entre la face supérieure P1 et le point P2 creux un tronçon 10" incurvé dont la concavité est tournée vers la diagonale P0P1q, la droite P1P2 étant inclinée par rapport à la verticale. Entre le point P sommet et le point P2 creux, la courbe 7" comporte un tronçon 9" incurvé ayant un point d'inflexion Pi. La droite PP2 est incliné par rapport à la verticale et l'horizontale.

A la figure 12, il est représenté un mode de réalisation dans lequel le point P1 et le point creux P2 sont confondus et le point P0 et P sont distincts, de sorte que la droite P0P existe mais la droite P1P2 n'existe pas (c'est à dire qu'elle n'est constituée que d'un simple point). La courbe 7 est constituée dans ce cas du segment rectiligne 8 qui correspond à P0P suivi du segment 9 qui correspond à PP1 ou PP2. Les deux segments font un angle entre eux et sont chacun incliné par rapport respectivement à la verticale et l'horizontale, c'est à dire que P0P est inclinés par rapport à la verticale et PP2 est incliné par rapport à l'horizontale.

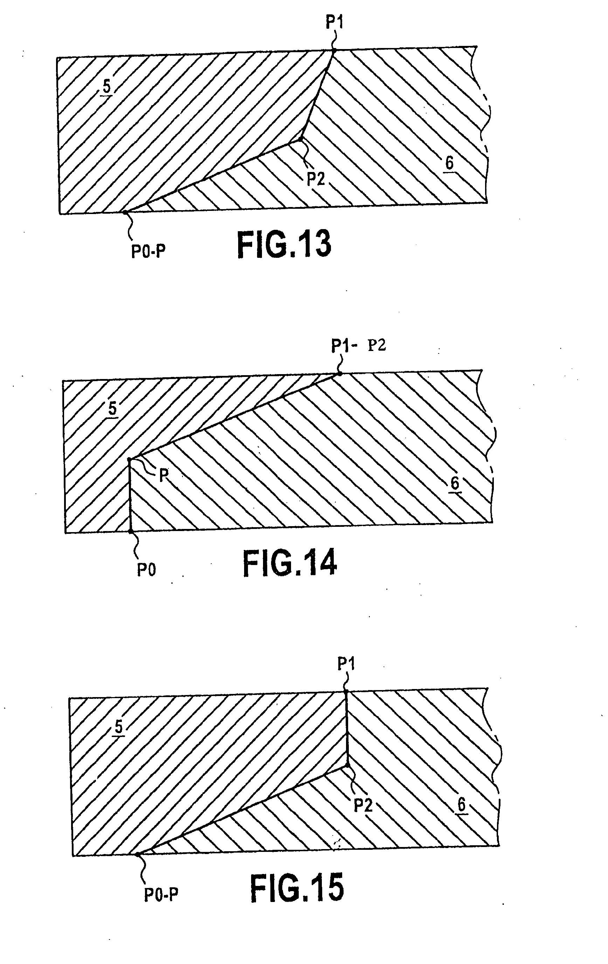

A la figure 13, il est représenté un mode de réalisation dans lequel le point P0 et le point creux P sont confondus et le point P1 et P2 sont distincts, de sorte que la droite P1P2 existe mais la droite P0P n'existe pas (c'est à dire qu'elle n'est constituée que d'un simple point). La courbe 7 est constituée dans ce cas du segment rectiligne 9 qui correspond à P0P2 suivi du segment 10 qui correspond à P1P2. Les deux segments font un angle entre eux et sont chacun inclinés par rapport respectivement à l'horizontale et la verticale, c'est à dire que P0P2 est incliné par rapport à l'horizontale et P1P2 est incliné par rapport à la verticale.

A la figure 14, il est représenté un mode de réalisation dans lequel le point P1 et le point creux P2 sont confondus et le point P0 et P sont distincts, de sorte que la droite P0P existe mais la droite P1P2 n'existe pas (c'est à dire qu'elle n'est constituée que d'un simple point). La courbe 7 est constituée dans ce cas du segment rectiligne 8 qui correspond à P0P suivi du segment 9 qui correspond à PP1 ou PP2. Les deux segments font un angle entre eux. P0P est vertical et PP1 ou PP2 est incliné par rapport à l'horizontale.

A la figure 15,il est représenté un mode de réalisation dans lequel le point P0 et le point creux P sont confondus et le point P1 et P2 sont distincts, de sorte que la droite P1P2 existe mais la droite P0P n'existe pas (c'est à dire qu'elle n'est constituée que d'un simple point). La courbe 7 est constituée dans ce cas du segment rectiligne 9 qui correspond à P0P2 suivi du segment 10 qui correspond à P1P2. Les deux segments font un angle entre eux. P0P2 est incliné par rapport à l'horizontale et P1P2 est vertical.

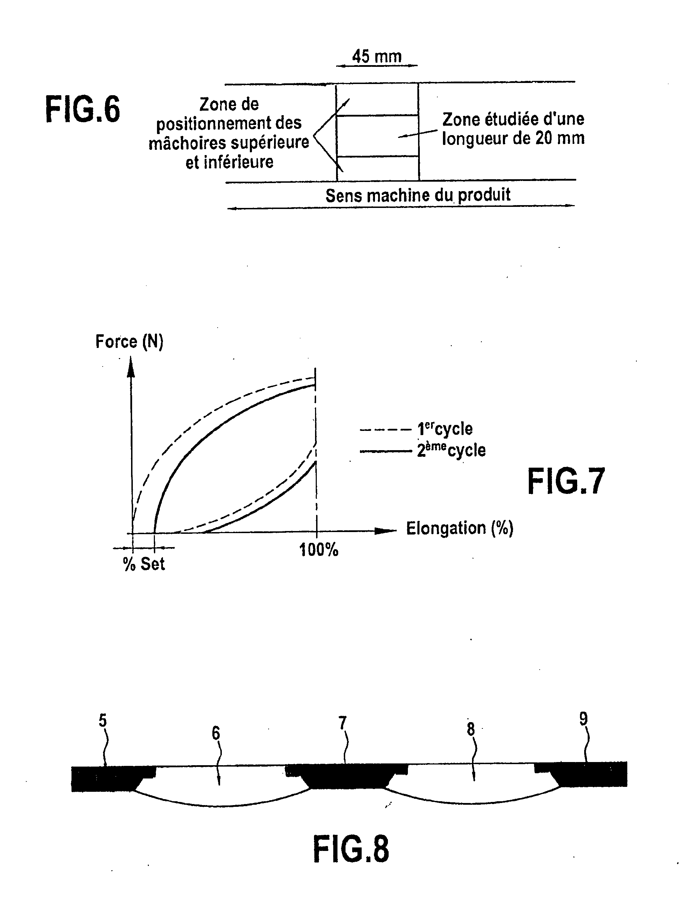

Comme on peut le voir par exemple à la figure 8, l'épaisseur de la strate mesurée dans la direction verticale à la figure, c'est-à-dire perpendiculairement à la direction en largeur et à la direction en longueur de défilement est sensiblement constante sur l'étendue transversale de chaque zone élastique ou de renforcement. Cependant, comme on peut le voir sur cette même figure 8, il peut arriver que l'épaisseur des zones élastiques varie de manière croissante puis décroissante en passant par un maximum entre ses deux jonctions avec les zones de renforcement, mais les épaisseurs du ou des film(s) élastique(s) et de la ou des zone(s) de renforcement sont telles qu'au niveau de leur jonction respective, les surfaces supérieure et/ou inférieure de la strate sont sensiblement sans aspérité ni discontinuité.

Ainsi par exemple, le ou les films élastiques ont un grammage environ 10 à 40%, de préférence 10 à 25% supérieur à celui du ou des films de renforcement, le ou les films élastiques ayant par exemple un grammage de 55 g/m² tandis que le ou les films de renforcement ont un grammage de 45 g/m².

Sur le tronçon de jonction ou liaison, qui s'étend sur une largeur en général comprise entre 3 et 10 mm, notamment 5mm, l'épaisseur de chaque film (de renforcement et élastique) est inférieure à l'épaisseur du film en dehors de la jonction. Ainsi, chaque film de la jonction comporte une partie de plus petite épaisseur qui vient s'assembler en superposition avec la partie de plus petite épaisseur de l'autre film de la jonction.

Dans la présente invention, on entend par film ou zone élastique dans une direction donnée un film ou une zone qui reprend sensiblement sa forme initiale après avoir été étiré ou allongé dans la direction donnée, à la température ambiante. De préférence il s'agit d'un film ou d'une zone qui ne conserve qu'une petite déformation résiduelle ou rémanence après déformation et relâchement (permanent set ou SET), à savoir inférieure à 10 %, plus préférablement inférieure à 5 % de la longueur initiale, pour un allongement de 100 % de la longueur initiale de l'échantillon, à température ambiante (T≈23°C).

Plus préférablement, le film élastique est capable de subir un allongement à la rupture d'au moins 300 % à température ambiante et, de préférence, un allongement d'au moins 600 %, et plus préférablement d'au moins 1 000 %, à température ambiante et pour une vitesse d'étirement de 508 mm/min. Le matériau en lequel est le film élastique peut être soit un élastomère pur, soit des mélanges ayant une phase élastomérique ou une matière qui présente toujours des propriétés sensiblement élastomériques à la température ambiante, comme par exemple des polyoléfines issus de catalyse type métallocène. Il peut également comporter une charge de matière thermoplastique pour entre autre aider à la formation de la strate d'un seul tenant avec les films de renforcements.

Pour mesurer la rémanence, ou SET, d'un échantillon, et donc déterminer s'il s'agit d'un échantillon d'un élément, notamment un film élastique, on utilise la méthode suivante :

On conditionne l'échantillon dans une atmosphère normale, telle que définie dans la norme ASTDM 5170, température de 23°C ± 2°C et humidité relative de 50 % ± 5 %.

On utilise comme appareillage un dynamomètre conforme à la norme EN 10002, notamment le Synergie 200H, 1 colonne disponible auprès de la société MTS Systems Corp, U.S.A., conjointement avec un logiciel d'utilisation TESTWORKS 4.04 B.

On prépare l'échantillon en découpant à l'emporte-pièce le produit dont on souhaite mesurer l'élasticité en un échantillon de forme rectangulaire (par exemple de 45 mm de largeur dans le sens MD (direction de la machine, perpendiculairement au plan de la Figure 1) et d'une longueur dans le sens CD (direction transversale, direction horizontale à la Figure 1) de 60 mm).

On positionne l'échantillon entre les mâchoires de part et d'autre de la zone dont on souhaite tester l'élasticité et de chaque côté, comme représenté schématiquement à la Figure 6.

Les paramètres sont sélectionnés comme suit :

| | |
|---|---|
| Distance inter mâchoires | : 20 mm |
| Vitesse machine | : 254 mm/min |
| Nombre de cycles | : 2 |
| Allongement du produit | : 100 % à vitesse constante |

On étire le produit à 100 % par déplacement vertical des mâchoires, après application d'une précharge de l'ordre de 0,05 N, puis on le maintient dans la position pendant 30 secondes (premier cycle), puis on le laisse revenir à la position initiale où on le laisse 60 secondes, puis on l'étire de nouveau à 100 %, on le maintient pendant 30 secondes (deuxième cycle) et on revient à la position initiale. On obtient alors la courbe donnant la force d'étirement en N en fonction de l'allongement en %, celle-ci présentant une hystérésis, qui peut alors être caractérisée par le SET (n=2), à savoir le point de la courbe de départ du deuxième cycle qui coupe l'axe des X (élongation en %) à la Figure 7.

Plus préférablement, le film élastique est capable de subir un allongement à la rupture d'au moins 300 % à température ambiante et, de préférence, un allongement d'au moins 600 %, et plus préférablement d'au moins 1 000 %, à température ambiante et pour une vitesse d'étirement de 508 mm/min. Le matériau en lequel est le film élastique peut être soit un élastomère pur, soit des mélanges ayant une phase élastomérique ou une matière qui présente toujours des propriétés sensiblement élastomériques à la température ambiante, comme par exemple des polyoléfines issus de catalyse type métallocène. Il peut également comporter une charge de matière thermoplastique pour entre autre aider à la formation de la strate d'un seul tenant avec les films de renforcements.

Le matériau du film élastique, notamment élastomérique, peut être un matériau élastique thermorétrécissable ou non thermorétrécissable. Il peut être formé, en particulier, à partir de polymères, tels que des copolymères de différents types de motif de monomères, par exemple alterné tel que A-B, ou séquencé, par exemple A-A-A-B-B-B, ou statistique, par exemple A-A-B-A-B-B-A-A-A-B-A, dont l'ensemble du réseau obtenu peut avoir différentes structures, soit linéaires de type A-B-A, soit radiales de type A-B, indice n (n>2), soit diblock de type A-B qui sont élastomériques, par exemple les copolymères styrène/isoprène (SI), styrène/isoprène/ styrène (SIS), styrène/butadiène/styrène (SBS), styrène-éthylène/butylène-styrène (SEBS), styrène-éthylène/ propylène-styrène (SEPS) ou SIBS. Des mélanges de ces élastomères les uns avec les autres ou avec des non élastomères modifiant certaines caractéristiques autres que l'élasticité peuvent également être pris en compte. Par exemple jusqu'à 50 % en poids mais, de préférence, moins de 30 % en poids de polymère peuvent être ajoutés en tant qu'ajout raidisseur, tels que des polyvinyles styrène, des polystyrènes ou des poly α-méthyl-styrène, polyesters époxy, polyoléfines, par exemple des polyéthylènes ou certains acétates d'éthylène/vinyle, de préférence ceux de poids moléculaire élevé.

Le matériau du film élastique peut être, notamment, un styrène-isoprène-styrène, disponible par exemple auprès de la Société Kraton Polymers, sous la dénomination KRATON D(Marque déposée), ou de la société DEXCO POLYMERS LP sous la dénomination VECTOR SBC 4211 (Marque déposée). On peut également utiliser un élastomère thermoplastique de polyuréthane, notamment le PELLATHANE (Marque déposée) 2102-75A de la Société The Dow Chemical Company. On peut utiliser également un styrène-butadiène-styrène, notamment le KRATON D-2122 (Marque déposée) de la société Kraton Polymers, ou le VECTOR SBC 4461 (Marque déposée)de la société Dexco Polymers LP. On peut aussi utiliser un styrène-éthylène/butylène, notamment le KRATON G-2832 (Marque déposée) de la société Kraton Polymers, ou un copolymère séquencé styrène-éthylène-butylène-styrène (SEBS), notamment le KRATON (Marque déposée) G2703. On peut également utiliser un copolymère d'acrylate d'isooctyle et d'acide acrylique suivant des rapports de monomère de 90/10. On peut également utiliser un copolymère séquencé polyamide polyéther PEBAX (Marque déposée) 2533 de la société Arkema.

D'autres matériaux possibles sont des polymères polyoléfines ayant les caractéristiques des élastomères, notamment issus de la catalyse métallocène, tel le VISTAMAXX VM-1120 (Marque déposée), disponible auprès de la société Exxon Mobil Chemical ou des polymères chargés EPDM du type Santoprène.

Dans le cas où de la colle est utilisée comme adhésif pour fixer les films élastiques et les films thermoplastiques aux non-tissés et éventuellement les non-tissés entre eux, on peut, suivant l'invention, utiliser une colle telle que des colles hot melt non réactive, par exemple la H2511 de Bostick, ou une colle réactive, notamment la AX75E de Bostik, ou des colles PU réactives.

De préférence, ces colles auront une nature chimique similaire ou compatible avec une matière composant le film élastique et/ou le film thermoplastique ou de renforcement décrit ci dessus.

Concernant les non-tissés, on peut utiliser des non-tissés de type spunbond et/ou meltblown et/ou cardé calandré et/ou spunlace et ayant comme matériau de base un ou des polyoléfines, de préférence du polypropylène, du polyester ou un mélange de ceux ci. En particulier, le non-tissé "spunbond" est à base de fibres longues ou filaments, ayant un titre en dTex (masse en gramme divisée par une longueur de 10 000 m de fil) compris entre 1 dTex et 6 dTex, par exemple 2,2 dTex. En particulier lorsque le stratifié comporte deux couches de non-tissé, les couches peuvent être de types différents et/ou de matériaux différents.

De préférence le ou au moins l'un des non-tissés ou les deux non-tissés a(ont) un grammage compris entre 5 et 25 g/m2, notamment entre 5 et 15 g/m2, plus particulièrement entre 5 et 10 g/m2.

En particulier, lorsque le non-tissé est un non-tissé qui n'a pas de capacité à s'étirer de soi-même et doit être activé, le grammage est compris entre 5 et 20 g/m², notamment il est d'environ 15 g/m² pour un spunbond ou d'environ 8 g/m² pour un meltblown. Lorsque le non-tissé n'a pas besoin d'être activé pour que le stratifié ait des capacités élastiques, c'est-à-dire lorsque le non-tissé a des capacités à s'étirer de lui-même sur un domaine d'allongement allant au moins jusqu'à 50 %, de préférence au moins 100 %, le grammage est de préférence compris entre 15 et 25 g/m², par exemple il est de 22 g/m² pour un cardé calandré ou de 25 g/m² pour un spunlace. En outre, il est à noter que les deux non-tissés, dans le cas où il y en a deux, peuvent être différents l'un de l'autre.

Pour déterminer si un élément, notamment un film, est plus étirable qu'un autre élément, par exemple un autre film, suivant l'invention, on peut utiliser un test d'allongement sous effort jusqu'à la rupture comme suit :

On utilise comme appareillage un dynamomètre conforme à la norme EN 10002, par exemple le même que pour le test d'élasticité ci-dessus, à savoir le Synergie 200H, 1 colonne, disponible auprès de la société MTS Systems Corp, USA, conjointement avec un logiciel d'utilisation TESTWORKS 4.04 B. On prépare l'échantillon en forme rectangulaire, par exemple de 45 mm de largeur et d'une longueur de 60 mm. On positionne l'échantillon entre les mâchoires de part et d'autre de la zone dont on souhaite tester l'allongement et de chaque côté, comme représenté schématiquement à la Figure 6.

Les paramètres sont sélectionnés comme suit :

| | |
|---|---|
| Distance inter mâchoires | : 20 mm |
| Vitesse machine | : 254 mm/min |
| Allongement du produit | : jusqu'à rupture à vitesse constante |

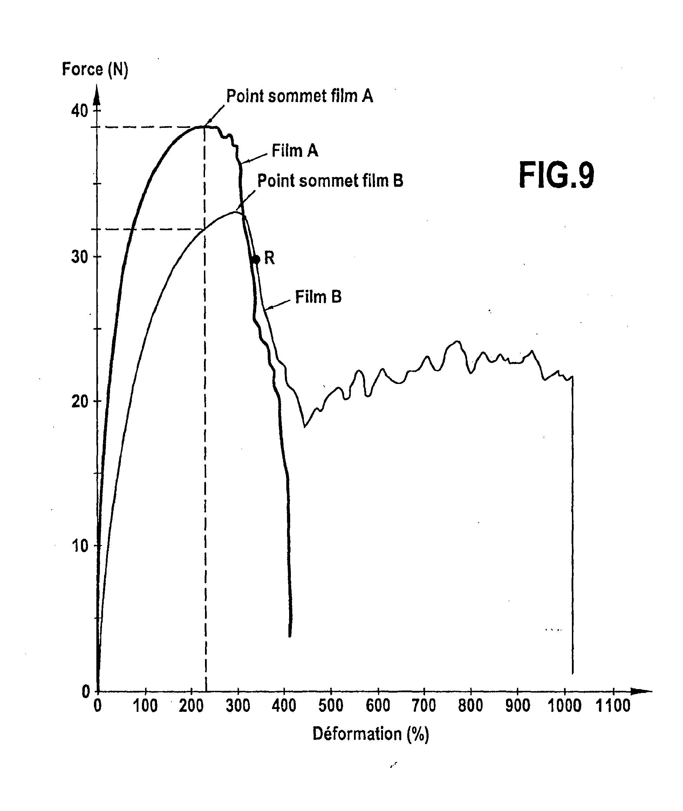

Après avoir appliqué une précharge de 0,05 N, on étire le produit jusqu'à sa rupture par déplacement vertical des mâchoires. On obtient alors pour chaque échantillon la courbe caractéristique donnant la force d'étirement en N en fonction de l'allongement en %, comme représenté à la Figure 9. Chaque courbe comporte un point sommet, à partir duquel l'allongement croît à force décroissante. Ce sommet correspond au passage à l'état de ruine de l'élément.

Pour déterminer lequel de deux éléments, notamment de deux films, est le moins étirable, on teste avec la méthode ci-dessus les deux éléments, notamment les deux films, de manière à obtenir leur courbe caractéristique respective. Chaque courbe présente un point sommet donné auquel correspond un allongement donné respectif. On choisit alors comme allongement de référence le plus petit des deux allongements donnés des deux courbes ou l'allongement donné dans le cas où les deux allongements donnés sont identiques et pour cet allongement donné le plus petit on compare les deux forces d'étirement de chaque courbe et l'élément ayant pour cet allongement de référence la force d'étirement la plus grande est l'élément le moins étirable. Ainsi, par exemple à la Figure 9, l'allongement de référence est l'allongement correspondant au point sommet du film A, à savoir environ 240 % et à cet allongement de référence, la courbe du film A donne une force d'étirement d'environ 40 N et la courbe du film B une force d'étirement d'environ 32,5 N, de sorte que c'est le film A qui est le moins étirable.

Concernant le matériau des bandes de renforcement, on peut choisir une matière ou un mélange de plusieurs matières telles que celles de la famille des thermoplastiques, par exemple le polyéthylène, le polypropylène, le polyester ou toute matière analogue, ainsi que des matières de la famille des élastomères thermoplastiques utilisées pour le film élastique (mais cependant de sorte que le film de renforcement soit dans tous les cas de moindre allongement que le film élastique), ce qui permet d'assurer une bonne compatibilité des matériaux venant former la strate d'un seul tenant. On choisira de préférence un matériau thermoplastique ayant une bonne compatibilité avec le matériau élastique ou élastomérique du film élastique pour que la liaison entre eux le long de leur bord respectif, après refroidissement, soit la plus forte possible. Des exemples précis de matière pour les films de renforcement sont le polyéthylène Riblène MV 10 (marque déposée) de la société Polimeri Europa, ou le MG 9621 de la société Boréalis, ou le LD259 ou LD655 de la société Exxon Mobil Chemical.

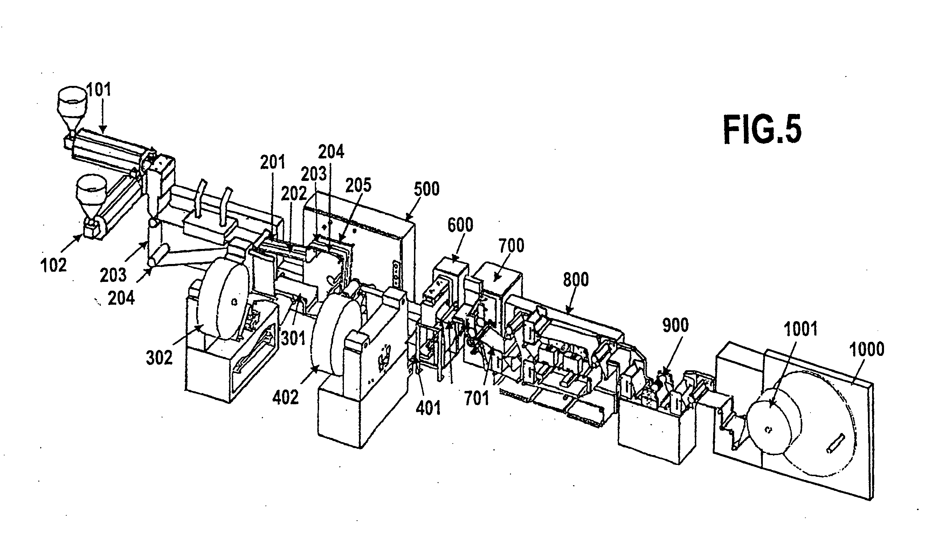

A la figure 5, il est représenté une installation de fabrication d'un stratifié suivant l'invention: l'installation comporte une extrudeuse 101 qui forme par extrusion deux rubans 201, 202 de films élastiques qui par l'intermédiaire de systèmes à courroies 203 et galets 204 sont transportés entre deux rouleaux en même temps que deux couches de non tissé 301 et 401 pour y être fixés par refroidissement de la matière élastomérique entre les non tissés dans une unité de fixation 500. Le non tissé 301 est déroulé à partir d'une bobine 302. Le deuxième non tissé 401 est déroulé d'une bobine 402. De l'extrudeuse 102 sont également formés trois rubans 203, 204, 205 en matière thermoplastique. Les cinq rubans 201 à 205 sont co-extrudés les uns à côté des autres en ayant leur bords contigus, de sorte qu'en sortie d'outillage de co-extrusion et par la suite après refroidissement, ils ne forment plus qu'une unique pièce. Le complexe constitué des non tissés 301 et 401 et des rubans élastiques et thermoplastiques 201 à 205, est à savoir le complexe 601, est ensuite laminé dans une calandre 600, découpé à la bonne largeur dans un système de découpe 700 en largeur comportant des couteaux 701 circulaires, puis passe dans une unité de soudure 800, permettant une soudure des bords longitudinaux. Les non tissés sont enfin activés par défibrillation, dans une unité 900 d'activation.

A la sortie de l'unité 900 d'activation, le stratifié dans son ensemble est enroulé sur un enrouleur 1000 en un rouleau 1001 qui pourra ensuite être amené directement sur les unités de fabrication des couches culottes pour la dépose sur une couche culotte.

De préférence, le ou les films élastiques ou élastomériques ont une épaisseur comprise entre 20 microns et 120 microns. De même les bandes de renforcement ont de préférence la même épaisseur.

Suivant les applications, on peut choisir les dimensions des films de renforcement en matière thermoplastique de sorte que l'une d'entre elle s'étende exactement jusqu'à un des bords gauche ou droit du stratifié, tandis qu'à l'autre bord, soit la bande s'étend également jusqu'au bord droit ou gauche exactement, soit dépasse d'une petite distance à l'extérieur au delà du bord du stratifié, soit au contraire se termine en retrait de ce bord de manière à former une petite lisière, notamment sans film de renforcement.

En particulier, pour obtenir la forme souhaitée de la courbe interface, outre sur la forme de l'outillage, on peut jouer sur les viscosités relatives des matériaux utilisés pour la réalisation de la partie rigide et de la partie élastique qui auront une conséquence sur les pressions intrinsèques respectives des deux matières en compétition lors de la création de la jonction. Ainsi, à outillages identiques, plus la viscosité relative du matériau de la partie élastique sera faible vis à vis du matériau de la partie rigide, plus la partie rigide en superposition avec la partie élastique sera de petite épaisseur.

Pour atteindre cet objectif, on utilise par exemple un matériau de viscosité de la partie élastique 20 à 50% plus faible que celui de la partie rigide, comme par exemple un matériau de MFR 3,15 g/lOmn pour la partie élastique et un matériau de MFR 4,60 g/10mn pour la partie rigide, MFR étant mesuré suivant la norme NF EN ISO 1133 à 190°C et 2,16 kg.

L'angle dont est incliné le premier segment rectiligne par rapport à la verticale peut notamment être compris entre 5 et 60°, de préférence entre 15 et 45°, notamment entre 25 et 40°.

L'angle entre la verticale et le deuxième segment peut être compris entre 30° et 90° (90° lorsqu'il est horizontal), notamment entre 60° et 90°, plus préférablement entre 75° et 90°.

Dans la présente invention, on a décrit le stratifié en définissant une face inférieure et une face supérieure. Cependant, il convient de noter que ceci est fait dans le but uniquement de simplifier la description et qu'inférieur et supérieur sont utilisés en liaison avec les figures et que dans la réalité leur position relative (en haut et en bas) peuvent être inversée sans sortir du domaine de protection de l'invention. Une autre manière plus précise, mais cependant plus ardue à la compréhension, de définir les choses eût pu consister à mentionner une face et une face opposée.

A la figure 15, il est représenté une photographie d'une vue en coupe transversale d'un stratifié suivant l'invention, notamment correspondant sensiblement au cas de la figure 1. La jonction est indiquée par un trait en pointillés rajouté.

## Revendications

1. stratifié comportant un empilement dans une direction verticale d'au moins une couche de non-tissé et d'une strate d'une pièce ou d'un seul tenant fixée à la au moins une couche de non-tissé, notamment par un adhésif tel que de la colle, la strate ayant une première face ou face inférieure et une deuxième face ou face supérieure entre lesquelles s'étendent au moins une zone élastique en forme de film et au moins une zone dite rigide en forme de film en un matériau moins élastique que le matériau de la zone élastique, notamment une zone en un matériau non élastique, la strate étant obtenue par coextrusion des au moins deux films élastique et rigide, de sorte qu'il est formé entre eux une interface, est **caractérisé en ce que**, vu en coupe,
- la courbe ou ligne formée par l'interface entre les deux films s'étend d'un point (P0) de la face inférieure à un point (P1) de la face supérieure mutuellement décalés dans la direction horizontale, la droite diagonale passant par ces deux points étant incliné par rapport à la verticale à la strate ;
- on définit le point (P) sommet de la courbe du côté du film rigide comme étant le point le plus éloigné de la droite diagonale du côté du film rigide, la distance à la droite diagonale étant mesurée perpendiculairement à celle ci, le point sommet étant confondu avec le point de la face inférieure dans le cas limite où la courbe ne dépasse pas la diagonale du côté du film rigide et on définit le point creux (P2) de la courbe du côté du film élastique comme étant le point le plus éloigné de la droite diagonale du côté du film élastique, la distance à la droite diagonale étant mesurée perpendiculairement à celle ci, le point creux étant confondu avec le point de la face supérieure dans le cas limite où la courbe ne dépasse pas la diagonale du côté du film élastique ;
- au moins l'un des points sommet et creux étant distincts respectivement des points inférieur et supérieur ;
- la droite passant par le point de la face inférieure et le point sommet et/ou la droite passant par le point de la face supérieure et le point creux faisant un angle avec la droite passant par le point creux et le point sommet ; et
- au moins l'une de la droite (P0P) point inférieur-point sommet, de la droite (PP2) point sommet-point creux et de la droite (P2P1) point creux-point supérieur est inclinée respectivement par rapport à la direction verticale, par rapport à la direction horizontale et par rapport à la direction verticale.

2. Stratifié suivant la revendication 1, **caractérisé en ce que** le point inférieur et le point sommet sont distincts, la droite point inférieur-point sommet est inclinée par rapport à la verticale et la droite point creux-point sommet est sensiblement horizontale.

3. Stratifié suivant la revendication 1, **caractérisé en ce que** le point inférieur et le point sommet sont distincts, la droite point inférieur point sommet est sensiblement verticale et la droite point sommet-point creux est inclinée par rapport à l'horizontale.

4. Stratifié suivant la revendication 1 ou 2, **caractérisé en ce que** le point inférieur et le point sommet sont distincts, la droite point inférieur-point sommet est inclinée par rapport à la verticale et la droite point sommet-point creux est inclinée par rapport à l'horizontale.

5. Stratifié suivant l'une des revendications précédentes, **caractérisé en ce que** la courbe (7, 7') comporte entre la face inférieure et le point sommet un segment (8, 8') inférieur sensiblement rectiligne, notamment rectiligne.

6. Stratifié suivant l'une des revendications précédentes, **caractérisé en ce que** la courbe (7, 7') comporte un segment (9, 9') intermédiaire qui s'étend du point sommet jusqu'au point creux.

7. Stratifié suivant l'une des revendications précédentes, **caractérisé en ce que** l'extension dans la direction horizontale de la droite point inférieur-point sommet est inférieure à l'extension dans la direction horizontale de la droite point sommet-point creux.

8. Stratifié suivant l'une des revendications précédentes, **caractérisé en ce que** le point creux est distinct du point supérieur; et la droite point supérieur point creux fait un angle avec la droite point sommet-point creux.

9. Stratifié suivant la revendication 8, **caractérisé en ce que** la courbe comporte un segment rectiligne qui s'étend entre le point supérieur et le point creux, notamment du point supérieur jusqu'au point creux.

10. Stratifié suivant l'une des revendications précédentes, **caractérisé en ce que** le point sommet se trouve à une hauteur par rapport à la face inférieure de la strate qui correspond à au moins 80 % de la hauteur totale ou épaisseur de la strate, telle que mesurée le long de la verticale passant par ce point.

## Patentansprüche

1. Schichtstoff, der in einer vertikalen Richtung einen Stapel von mindestens einer Vliesstoffschicht und einer Lage aus einem Teil oder einem Stück, die an der mindestens einen Vliesstoffschicht befestigt ist, insbesondere durch ein Klebemittel wie einen Klebstoff, aufweist, wobei die Lage eine erste Seite oder Unterseite und eine zweite Seite oder Oberseite aufweist, zwischen denen sich mindestens eine elastische Zone in Form einer Folienschicht und mindestens eine sogenannte starre Zone in Form einer Folienschicht aus einem weniger elastischen Material als das Material der elastischen Zone, insbesondere eine Zone aus einem nichtelastischen Material, erstrecken, wobei die Lage durch Koextrusion der mindestens zwei Folienschichten, der elastischen und der starren, derart erhalten wird, dass zwischen diesen eine Grenzfläche ausgebildet wird, **dadurch gekennzeichnet, dass**, im Querschnitt betrachtet,
- die Kurve oder Linie, die durch die Grenzfläche zwischen den beiden Folienschichten gebildet ist, sich von einem Punkt (P0) der Unterseite zu einem Punkt (P1) der Oberseite, die in der horizontalen Richtung gegenseitig verschoben sind, erstreckt, wobei die durch diese beiden Punkte hindurchgehende Diagonale in Bezug auf die Vertikale zu der Lage geneigt ist,
- der Gipfelpunkt (P) der Kurve der Seite der starren Folienschicht als der Punkt definiert ist, der von der Diagonalen auf der Seite der starren Folienschicht am weitesten entfernt ist, wobei der Abstand zur Diagonalen senkrecht zu dieser gemessen wird, wobei der Gipfelpunkt in dem Grenzfall, in dem die Kurve nicht von der Diagonalen auf der Seite des starren Films abweicht, mit dem Punkt der Unterseite verschmolzen ist, und der Muldenpunkt (P2) der Kurve auf der Seite der elastischen Folienschicht als der Punkt definiert ist, der von der Diagonalen auf der Seite des elastischen Films am weitesten entfernt ist, wobei der Abstand zur Diagonalen senkrecht zu dieser gemessen wird, wobei der Muldenpunkt in dem Grenzfall, in dem die Kurve nicht von der Diagonalen auf der Seite des elastischen Films abweicht, mit dem Punkt der Oberseite verschmolzen ist,
- mindestens einer von dem Gipfelpunkt und dem Muldenpunkt von dem oberen Punkt bzw. dem unteren Punkt verschieden ist,
- die Gerade, die durch den Punkt der Unterseite und den Gipfelpunkt hindurchgeht, und/oder die Gerade, die durch den Punkt der Oberseite und den Muldenpunkt hindurchgeht, einen Winkel mit der Geraden, die durch den Muldenpunkt und den Gipfelpunkt hindurchgeht, bilden, und
- mindestens eine von der Geraden (P0P) unterer Punkt-Gipfelpunkt, der Geraden (PP2) Gipfelpunkt-Muldenpunkt und der Geraden (P2P1) Muldenpunkt-oberer Punkt in Bezug auf die vertikale Richtung, in Bezug auf die horizontale Richtung bzw. in Bezug auf die vertikale Richtung geneigt ist.

2. Schichtstoff nach Anspruch 1, **dadurch gekennzeichnet, dass** der untere Punkt und der Gipfelpunkt verschieden sind, die Gerade unterer Punkt-Gipfelpunkt in Bezug auf die Vertikale geneigt ist und die Gerade Muldenpunkt-Gipfelpunkt im Wesentlichen horizontal ist.

3. Schichtstoff nach Anspruch 1, **dadurch gekennzeichnet, dass** der untere Punkt und der Gipfelpunkt verschieden sind, die Gerade unterer Punkt-Gipfelpunkt in Wesentlichen vertikal ist und die Gerade Gipfelpunkt-Muldenpunkt in Bezug auf die Horizontale geneigt ist.

4. Schichtstoff nach Anspruch 1 oder 2, **dadurch gekennzeichnet, dass** der untere Punkt und der Gipfelpunkt verschieden sind, die Gerade unterer Punkt-Gipfelpunkt in Bezug auf die Vertikale geneigt ist und die Gerade Gipfelpunkt-Muldenpunkt in Bezug auf die Horizontale geneigt ist.

5. Schichtstoff nach einem der vorhergehenden Ansprüche, **dadurch gekennzeichnet, dass** die Kurve (7, 7') zwischen der Unterseite und dem Gipfelpunkt ein unteres Segment (8, 8') aufweist, das im Wesentlichen geradlinig, insbesondere geradlinig ist.

6. Schichtstoff nach einem der vorhergehenden Ansprüche, **dadurch gekennzeichnet, dass** die Kurve (7, 7') ein mittleres Segment (9, 9') aufweist, das sich vom Gipfelpunkt bis zum Muldenpunkt erstreckt.

7. Schichtstoff nach einem der vorhergehenden Ansprüche, **dadurch gekennzeichnet, dass** die Erstreckung der Geraden unterer Punkt-Gipfelpunkt in der horizontalen Richtung geringer als die Erstreckung der Geraden Gipfelpunkt-Muldenpunkt in der horizontalen Richtung ist.

8. Schichtstoff nach einem der vorhergehenden Ansprüche, **dadurch gekennzeichnet, dass** der Muldenpunkt vom oberen Punkt verschieden ist und die Gerade oberer Punkt-Muldenpunkt einen Winkel mit der Geraden Gipfelpunkt-Muldenpunkt bildet.

9. Schichtstoff nach Anspruch 8, **dadurch gekennzeichnet, dass** die Kurve ein geradliniges Segment aufweist, das sich zwischen dem oberen Punkt und dem Muldenpunkt, insbesondere vom oberen Punkt bis zum Muldenpunkt erstreckt.

10. Schichtstoff nach einem der vorhergehenden Ansprüche, **dadurch gekennzeichnet, dass** sich der Gipfelpunkt auf einer Höhe in Bezug auf die Unterseite der Lage befindet, die mindestens 80% der Gesamthöhe oder Dicke der Lage entspricht, die entlang der durch diesen Punkt hindurchgehenden Vertikalen gemessen wird.

## Claims

1. Laminate comprising a stack, in a vertical direction, of at least one non-woven layer and a layer made in one part or as an integral part attached to the at least one non-woven layer, in particular by means of an adhesive such as glue, the layer comprising a first face or lower face and a second face or upper face between which at least one elastic section in the form of a film extends and at least one rigid section in the form of a film made from a less elastic material than the material of the elastic section, in particular a section made from a non-elastic material, the layer being obtained by coextrusion of the at least two elastic and rigid films, such that an interface is formed between them, **characterised in that**, as viewed in cross section,
- the curve or line formed by the interface between the two films extends from a point (P0) of the lower face to a point (P1) of the upper face mutually offset in horizontal direction, the diagonal line passing through these two points being inclined in relation to the vertical of the layer;
- the peak point (P) of the curve from the side of the rigid film is defined as the point most remote from the diagonal line from the side of the rigid film, the distance from the diagonal line being measured perpendicular to the latter, the peak point being merged with the point of the lower face in cases where the curve does not exceed the diagonal from the side of the rigid film and the hollow point (P2) of the curve from the side of the elastic film is defined as being the point that is most remote from the diagonal line from the slide of the elastic film, the distance to the diagonal line being measure perpendicular to the latter, the hollow point being merged with the point of the upper face in cases where the curve does not exceed the diagonal from the side of the elastic film;
- at least one of the peak and hollow points being different respectively from the lower and upper points;
- the line passing through the point of the lower face and the peak point and/or the line passing through the point of the upper face and the hollow point forms an angle with the line passing through the hollow point and the peak point; and
- at least one out of the line (P0P) lower point-peak point, the line (PP2) peak point-hollow point and line (P2P1) hollow point-upper point is inclined respectively in relation to the vertical direction, in relation to the horizontal direction and in relation to the vertical direction.

2. Laminate according to claim 1, **characterised in that** the lower point and the peak point are different, the line lower point-peak point is inclined in relation to the vertical and the line hollow point-peak point is substantially horizontal.

3. Laminate according to claim 1, **characterised in that** the lower point and the peak point are different, the line lower point-peak point is substantially vertical and the line peak point-hollow point is inclined in relation to the horizontal.

4. Laminate according to claim 1 or 2, **characterised in that** the lower point and the peak point are different, the line lower point-peak point is inclined in relation to the vertical and the line peak point-hollow point is inclined in relation to the horizontal.

5. Laminate according to any one of the preceding claims, **characterised in** the curve (7, 7') comprises between the lower face and the peak point a lower segment (8, 8') that is substantially straight, in particular is straight.

6. Laminate according to any one of the preceding claims, **characterised in that** the curve (7, 7') comprises an intermediate segment (9, 9') which extends from the peak point to the hollow point.

7. Laminate according to any one of the preceding claims, **characterised in that** the extension in horizontal direction of the line lower point- peak point is smaller than the extension in horizontal direction of the line peak point-hollow point.

8. Laminate according to any one of the preceding claims, **characterised in that** the hollow point is distinct from the upper point and the line upper point - hollow point forms an angle with the line peak point-hollow point.

9. Laminate according to claim 8, **characterised in that** the curve has a straight segment which extends between the upper point and the hollow point, in particular from the upper point to the hollow point.

10. Laminate according to any one of the preceding claims, **characterised in that** the peak point is located at a level relative to the lower face of the layer which corresponds to at least 80% of the total height or thickness of the layer, as measured along the vertical line passing through this point.
